(19) Europäisches Patentamt
European Patent Office
Office européen des brevets

(11) Veröffentlichungsnummer: **0 288 819 B1**

# (12) EUROPÄISCHE PATENTSCHRIFT

(45) Veröffentlichungstag der Patentschrift: **17.07.91**

(51) Int. Cl.⁵: **C07C 333/16**

(21) Anmeldenummer: **88105851.5**

(22) Anmeldetag: **13.04.88**

## (54) Verfahren zur Herstellung von Dialkyldithiocarbamaten mehrwertiger Metalle.

(30) Priorität: **30.04.87 DE 3714436**

(43) Veröffentlichungstag der Anmeldung:
**02.11.88 Patentblatt 88/44**

(45) Bekanntmachung des Hinweises auf die Patenterteilung:
**17.07.91 Patentblatt 91/29**

(84) Benannte Vertragsstaaten:
**AT BE CH DE ES FR GB GR IT LI LU NL SE**

(56) Entgegenhaltungen:
**DD-A- 253 816**
**DE-A- 2 635 511**
**DE-C- 1 094 729**
**GB-A- 1 487 968**

(73) Patentinhaber: **C.F. Spiess & Sohn GmbH & Co. Chemische Fabrik**

**W-6719 Kleinkarlbach(DE)**

(72) Erfinder: **Spiess, Wolfram, Dr.**
**Heinrich-Becker-Strasse 16**
**W-6718 Grünstadt(DE)**
Erfinder: **Himmelreich, Rolf**
**Uhlandstrasse 9**
**W-6718 Grünstadt(DE)**

(74) Vertreter: **Eggert, Hans-Gunther, Dr.**
**Räderscheidtstrasse 1**
**W-5000 Köln 41(DE)**

EP 0 288 819 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Dialkyl-dithiocarbamate der mehrwertigen Metalle werden im allgemeinen durch doppelte Umsetzung von Dialkyl-dithiocarbamaten der Alkalimetalle, des Ammoniums oder der Dialkylammoniumverbindungen mit Metallsalzen hergestellt. Siehe Elsevier Monographs "The Dithiocarbamates and related Compounds" von Thorn and Ludwig (1962).

Aus der GB-A-1 487 968 ist ein Verfahren zur Herstellung von Antimon-N-(2-octyl)-N-ethyldithiocarbamt bekannt, in dem N-(2-octyl)-N-ethylamin mit Antimontrioxid und Kohlendisulfid unter Rückfluß in Hexan umgesetzt wird. Nach einer heißen Filtration und Abkühlen des Gemischs wird das Hexan entfernt und die nach diesem Verfahren hergestellte Verbindung als Zusatz für Schmieröle bereitgestellt.

Es wurde nun gefunden, daß die Herstellung auch direkt durch Reaktion von sekundären Aminen mit Schwefelkohlenstoff und mehrwertigen Metalloxiden in stöchiometrischen Mengen in einem Mineralöl möglich ist. Dabei muß die Mischung auf Temperaturen von 50 bis 95 °C erwärmt werden , wobei sich eine den Sauerstoffatomen des Metalloxids entsprechende Menge Wasser bildet.

Durch dieses Verfahren werden die Metall-dialkyl-dithiocarbamate, die als Additive und Antioxidantien in Hydraulik- und Getriebeölen eingesetzt werden, direkt in dem Medium hergestellt, in dem sie als Additiv benötigt werden, was eine ganze Reihe von Vorteilen mit sich bringt.

Die erfindungsgemäße Herstellung der Metall-dialkyl-dithiocarbamate entspricht folgendem Reaktionsmechanismus:

Für zweiwertige Metalle:

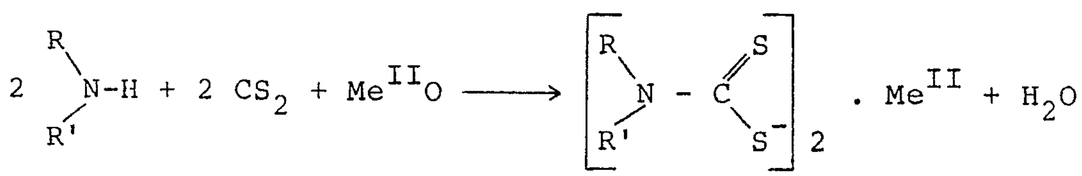

Für dreiwertige Metalle:

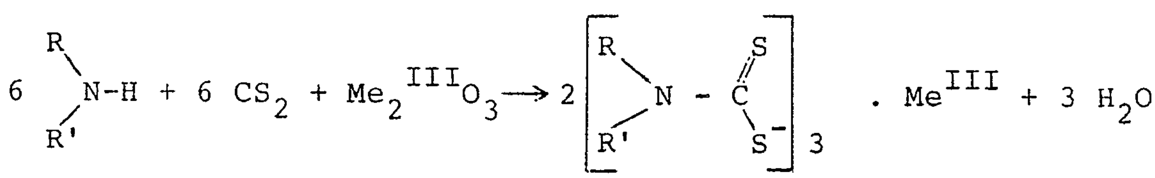

Für vierwertige Metalle:

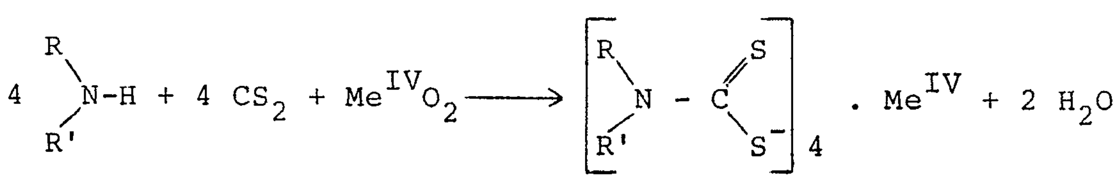

In den allgemeinen Formeln bedeuten R und R' gleiche oder verschiedene, verzweigte oder unverzweigte, gesättigte oder ungesättigte Alkyl- oder Cycloalkylreste mit 1 bis 13 Kohlenstoffatomen.

Beispiele für zweiwertige Metalloxide $Me^{II}O$ sind CuO, ZnO und PbO.

Beispiele für die Oxide dreiwertiger Metalle $Me_2^{III}O_3$ sind $Fe_2O_3$, $Ni_2O_3$ und $Sb_2O_3$.

Ein Beispiel für das Oxid eines vierwertigen Metalls $Me^{IV}O_2$, das nach dem erfindungsgemäßen Verfahren direkt in ein Dialkyldithiocarbamat überführt werden kann, ist Mangandioxid.

Wenn das Metall-dialkyl-dithiocarbamat unter den Reaktionsbedingungen flüssig ist, also noch nicht auskristallisiert, kann die Umsetzung ohne ein Lösungsmittel durchgeführt werden. Das Reaktionswasser wird dann nach im wesentlichen beendeter Umsetzung, vorzugsweise aber schon im Maße seiner Bildung während der Reaktion im Vakuum abdestilliert.

Die Herstellung der Metall-dialkal-dithiocarbamate erfolgt in einem hochsiedenden Mineralöl, das einen Aromatenanteil von 1 bis 15 % haben kann, wie z.B. Raffinaten, Weißölen oder Spindelölen mit Siedebeginn ab 250 °C bei 760 Torr. Anstelle der Mineralöle können auch hochsiedende Esterwachse oder Fettsäureester eingesetzt werden.

Mit Mineralöl als Lösungsmittel kann beispielsweise wie folgt gearbeitet werden:
Das Mineralöl wird vorgelegt und unter Rühren das feingepulverte Metalloxid und das sekundäre Amin zugegeben. Nach dem Zutropfen des Schwefelkohlenstoffs und einer Nachreaktionsdauer wird die Mischung dann unter mechanischem Rühren 1 - 12 Stunden lang am Rückflußkühler auf 50 - 95 °C erhitzt. Danach wird das entstandene Wasser im Vakuum abdestilliert. Man kann die Erwärmung aber auch im Vakuum durchführen, wobei das entstehende Wasser gleich abdestilliert wird, was die Reaktionsdauer verkürzt. Die verbleibende Lösung wird filtriert und man erhält direkt eine hochprozentige Lösung des Wirkstoffs im Mineralöl.

Im allgemeinen wird bei der erfindungsgemäßen Herstellung der Metall-dialkyl-dithiocarbamate ein stöchiometrischer Überschuss an Metalloxid von 10 bis 100 % eingesetzt. Manche Metalloxide, wie Bleioxid und Zinkoxid bilden bei der Reaktion in geringer Menge Metallsulfide, die der Reaktion nicht mehr zugänglich sind. Die ggf. Sulfid enthaltenden Metalloxid-Rückstände können in weiteren Ansätzen wiederverwendet werden. Bei Verwendung von Zinkoxid in 100%-igem Überschuß sieg der Zinksulfidgehalt z.B. von 6,6 % im Rückstand des ersten Ansatzes auf 25,6 % nach dem 8. Ansatz und blieb dann konstant.
Auch der Schwefelkohlenstoff wird wegen seiner hohen Flüchtigkeit am günstigsten mit einem stöchiometrischen Überschuss von 5 - 20 % eingesetzt.

Die erfindungemäße Herstellung der Metall-dialkyl-dithiocarbamate wird in den folgenden Beispielen näher beschrieben:

Beispiel 1: Blei-bis-(diamyl-dithiocarbamat).

111,5 g Blei-II-oxid (Merck) werden zu 300 g Solvent Raffinat HR 22 (Lehmann und Voss) gegeben. Dazu gibt man 157 g Diamylamin (Hoechst). Unter mechanischem Rühren am Rückflußkühler tropft man dazu im Verlauf von 30 Minuten 64 ml Schwefelkohlenstoff. Die Mischung wird noch 45 Minuten lang weiter gerührt. Dann wird mit absteigenden Kühler im Wasserstrahl-Vakuum 2 1/2 Stunden lang auf 90 °C erwärmt. Dabei wird das entstehende Reaktionswasser abdestilliert. Anschließend werden 2 g Filtrierhilfsmittel (Kieselgur) (Seitz) zugesetzt und die Lösung heiß abgesaugt. Es werden 600 g eines bernsteingelben Öls erhalten, das einen Gehalt von 52,1 % hat.

Die Gehaltsbestimmung erfolgt bei den hier beschriebenen Dithiocarbamaten über eine Stickstoffbestimmung oder über eine Abspaltung von Schwefelkohlenstoff, der dann quantitativ bestimmt wird.

Beispiel 2: Blei-bis-(diisononyl-dithiocarbamat).

In einen 10 Liter Dreihalskolben gibt man 1125 g Bleioxid, 3100 g Solvent Raffinat HR 22 und 2690 g Di-isononyl-amin (Hoechst). Unter mechanischem Rühren am Rückflußkühler tropft man dazu im Verlauf von einer Stunde 640 ml Schwefelkohlenstoff. Die Mischung wird 90 Minuten lang weiter gerührt. Dann wird die Mischung 9 Stunden lang im Vakuum auf 90 °C erwärmt. Anschließend wird, nach Zusatz von 10 g Filtrierhilfsmittel, durch eine mit Niederdruckdampf beheizte Nutsche abgesaugt. Es werden 6228 g eines hellbernsteinfarbenen Öls erhalten, das einen Gehalt von 55,6 % hat. Es werden noch 522 g Solvent Raffinat HR 22 zugegeben. Es werden nun 6750 g Öl erhalten, das 51 % des Bleisalzes enthält.

Beispiel 3: Zink-bis-(diamyl-dithiocarbamat).

100 g Zinkoxid werden mit 278,7 g Deumos Spezial Raffinat 32 (Texaco) und 196,3 g Diamylamin versetzt. Unter mechanischem Rühren am Rückflußkühler werden dazu im Verlauf von 30 Minuten 80 ml Schwefelkohlenstoff getropft. Es wird noch 30 Minuten lang weiter gerührt. Dann wird noch 3 Stunden lang im Wasserbad auf 95 °C erwärmt. Das entstandene Reaktionswasser wird anschliessend im Vakuum im Rotationsverdampfer abdestilliert. Vom Ungelösten wird abgesaugt. Es werden 575 g eines hellgelben Öls erhalten, das einen Gehalt von 52,8 % hat.

Beispiel 4: Zink-bis-(isobutyl-isooctyl-dithiocarbamat).

82 g Zinkoxid werden mit 295 g Solvent Raffinat HR 22 und 185 g Isobutyl-isooctylamin (Hoechst) versetzt. Unter mechanischem Rühren am Rückflußkühler tropft man dazu im Verlauf von 30 Minuten 64 ml Schwefelkohlenstoff. Die Mischung wird noch 30 Minuten lang weiter gerührt und dann 5 Stunden lang im Wasserbad auf 95 °C erwärmt. Im Vakuum im Rotationsverdampfer wird das gebildete Wasser abdestilliert.

Nach Zusatz von 3 g Kieselgur wird heiß abgesaugt. Es werden 503 g eines hellgelben Öls erhalten, das einen Gehalt von 51,1 % hat.

Beispiel 5: Kupfer-bis-(diamyl-dithiocarbamat).

160 g Kupferoxid werden mit 500 g Solvent Raffinat HR 22 und 314 g Diamylamin versetzt. Dazu tropft man unter mechanischem Rühren am Rückflußkühler im Verlauf von 30 Minuten 128 ml Schwefelkohlenstoff. Die Mischung wird 9 Stunden lang auf 95°C erwärmt. Im Vakuum im Rotationsverdampfer wird das entstandene Wasser abdestilliert. Anschließend wird die Lösung abgesaugt. Man erhält 952 g einer braunen, viskosen Flüssigkeit mit einem Gehalt von 50,2 %.

Beispiel 6: Eisen-tris-(diamyl-dithiocarbamat).

100 g Eisen-III-oxid werden mit 475 g Solvent Raffinat HR 22 und 314 g Diamylamin versetzt. Dazu tropft man unter mechanischem Rühren am Rückflußkühler im Verlauf von 30 Minuten 128 ml Schwefelkohlenstoff. Die Mischung wird 9 Stunden lang im Wasserbad auf 95°C erwärmt. Anschließend wird im Vakuum im Rotationsverdampfer bei einer Badtemperatur von 90°C das entstandene Wasser abdestilliert. Vom Ungelösten wird heiß abgesaugt. Beim Abkühlen kristallisiert das Eisensalz aus. Vom Niederschlag wird abgesaugt. Der Rückstand wird aus Isopropanol umkristallisiert. Es werden 388 g einer schwarzen, glänzenden, kristallinen Substanz mit einem Schmelzpunkt von 85°C erhalten. Das Produkt hat einen Gehalt von 98,1 %.

Beispiel 7: Antimon-tris-(diamyl-dithiocarbamat).

In einen 2000 Liter Email-Kessel gibt man 537 kg Spindelöl Raffinat ET 22 Iso (Ölwerke Julius Schindler, Hamburg), 90 kg Antimonoxid ($Sb_2O_3$) und 263 kg Diamylamin. Dazu werden unter Rühren 136 kg Schwefelkohlenstoff im Verlauf von 45 Minuten eingetragen. Die Mischung wird noch eine Stunde lang weiter gerührt. Dann wird im Vakuum erwärmt und dabei das Reaktionswasser abdestilliert. Bei 55°C läuft die Hauptreaktion ab. Nach 2 Stunden beginnt die destillierende Wassermenge abzunehmen und die Temperatur steigt weiter an. Die Mischung wird noch 3 Stunden lang auf 90 - 93°C erwärmt. Danach werden 2,5 kg Filtrierhilfsmittel (Kieselgur) zugesetzt und heiß durch eine Filterpresse abgesaugt. Man erhält 957 kg eines bernsteingelben Öls mit einem Gehalt von 45,2 %. Die Ausbeute beträgt 97,7 % der Theorie.

Beispiel 8: Mangan-tetra-(di-2-ethylhexyl-dithiocarbamat).

52 g Mangandioxid (85-90%-ig) werden mit 482 g Di-2-ethylhexyl-amin versetzt. Dazu tropft man unter mechanischem Rühren am Rückflußkühler im Verlauf von 40 Minuten 128 ml Schwefelkohlenstoff. Die Mischung wird 3 Stunden lang weiter gerührt. Dann wird im Rotationsverdampfer im Vakuum auf 95°C erwärmt. Die Reaktionsdauer beträgt 10 Stunden. Anschließend wird vom Ungelösten durch eine dampfbeheizte Nutsche abgesaugt. Es werden 573 g eines schwarzbraunen Öls erhalten, das einen Gehalt von 94,34 % hat. Die Ausbeute beträgt 86,6 % der Theorie.

**Patentansprüche**

1. Verfahren zur Herstellung von Dialkyldithiocarbamaten mehrwertiger Metalle, dadurch gekennzeichnet, daß man das Oxid eines mehrwertigen Metalls mit einem sekundären Amin und Schwefelkohlenstoff unter Erwärmen auf 50 bis 95°C und Entfernung des entstehenden Reaktionswassers in einem Mineralöl umsetzt.

2. Verfahren nach Anspruch 1, dadurch gekennzeichnet, daß Bleioxid, Zinkoxid, Kupfer-II-oxid, Eisen-III-oxid, Nickel-III-oxid, Antimon-III-oxid oder Mangandioxid als Metalloxid eingesetzt werden.

3. Verfahren nach Anspruch 1 oder 2, dadurch gekennzeichnet, daß das sekundäre Amin die allgemeine Formel

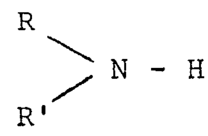

hat, in der R und R' gleiche oder verschiedene, verzweigte oder unverzweigte, gesättigte oder ungesättigte Alkyl- oder Cycloalkylreste mit 1 bis 13 Kohlenstoffatomen bedeuten.

4. Verfahren nach einem der Ansprüche 1 bis 3, dadurch gekennzeichnet, daß als Lösungsmittel Spindelöl, Weißöl, Raffinatöl, Esterwachse oder Fettsäureester, erforderlichenfalls in geschmolzener Form, eingesetzt werden.

5. Verfahren nach Anspruch 4, dadurch gekennzeichnet, daß das Reaktionswaser während der Reaktion im Vakuum abdestilliert wird.

6. Verfahren nach einem der Ansprüche 1 bis 5, dadurch gekennzeichnet, daß das Metalloxid in einem stöchiometrischen Überschuß von 10 bis 100 % eingesetzt wird.

7. Verfahren nach einem der Ansprüche 1 bis 6, dadurch gekennzeichnet, daß der Schwefelkohlenstoff in einem stöchiometrischen Überschuß von 5 bis 20 % eingesetzt wird.

**Claims**

1. Method for the preparation of dialkyldithiocarbamates of polyvalent metals, characterised in that the oxide of a polyvalent metal is reacted in a mineral oil with a secondary amine and carbon disulphide with heating to 50 to 95° C and removal of the resulting water of reaction.

2. Method according to Claim 1, characterised in that lead oxide, zinc oxide, copper (II) oxide, iron (III) oxide, nickel (III) oxide, antimony (III) oxide or manganese dioxide are used as the metal oxide.

3. Method according to Claim 1 or 2, characterised in that the secondary amine has the general formula

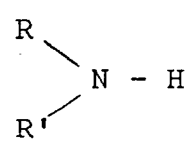

in which R and R' are identical or different, branched or unbranched, saturated or unsaturated alkyl or cycloalkyl radicals with 1 to 13 carbon atoms.

4. Method according to one of Claims 1 to 3, characterised in that spindle oil, white oil, refined oil, ester waxes or fatty acid esters, if necessary in molten form, are used as the solvent.

5. Method according to Claim 4, characterised in that the water of reaction is distilled off under vacuum during the reaction.

6. Method according to one of Claims 1 to 5, characterised in that the metal oxide is used in a stoichiometric excess of 10 to 100%.

7. Method according to one of Claims 1 to 6, characterised in that the carbon disulphide is used in a stoichiometric excess of 5 to 20%.

**Revendications**

1. Procédé de production de dialkyldithiocarbamates de métaux polyvalents, caractérisé en ce qu'on fait réagir dans une huile minérale l'oxyde d'un métal polyvalent avec une amine secondaire et le sulfure

de carbone en chauffant à 50-95 ° C et en éliminant l'eau de réaction produite.

2. Procédé suivant la revendication 1, caractérisé en ce qu'on utilise comme oxyde métallique l'oxyde de plomb, l'oxyde de zinc, l'oxyde de cuivre-II, l'oxyde de fer-III, l'oxyde de nickel-III, l'oxyde d'antimoine-III ou le bioxyde de manganèse.

3. Procédé suivant la revendication 1 ou 2, caractérisé en ce que l'amine secondaire répond à la formule générale

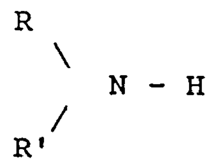

dans laquelle R et R' sont des restes alkyle ou cycloalkyle identiques ou différents, ramifiés ou non ramifiés, saturés ou non saturés ayant 1 à 13 atomes de carbone.

4. Procédé suivant l'une des revendications 1 à 3, caractérisé en ce qu'on utilise comme solvant l'huile pour broches, l'huile blanche, une huile raffinée, des cires d'esters ou des esters d'acides gras, en cas de besoin à l'état fondu.

5. Procédé suivant la revendication 4, caractérisé en ce que l'eau de réaction est chassée pendant la réaction par distillation sous vide.

6. Procédé suivant l'une des revendications 1 à 5, caractérisé en ce que l'oxyde métallique est utilisé en un excès stoechiométrique de 10 à 100 %.

7. Procédé suivant l'une des revendications 1 à 6, caractérisé en ce qu'on utilise le sulfure de carbone en un excès stoechiométrique de 5 à 20 %.